# EUROPEAN PATENT SPECIFICATION

(11) **EP 3 335 718 B1**
(45) Date of publication and mention of the grant of the patent: **02.12.2020**
(21) Application number: 15900836.6
(22) Date of filing: 31.08.2015
(51) Int. Cl.: A61K 36/899, A61K 36/752, A61K 36/734, A61K 36/62, A61K 36/605, A61K 36/288, A61K 36/00, A61P 39/02, A61K 36/888

(54) **CHINESE MEDICINE HANGOVER CURING AGENT**
MITTEL AUS DER CHINESISCHEN MEDIZIN GEGEN KATER
AGENT DE TRAITEMENT DE LA GUEULE DE BOIS EN MÉDECINE CHINOISE

(30) Priority: 10.08.2015 CN 201510487918
(43) Date of publication of application: 20.06.2018
(73) Proprietor: Wang, Lei, Beijing 100069 (CN)
(72) Inventor: LI, E, Beijing 100069 (CN)
(74) Representative: Lösch, Christoph Ludwig Klaus
(86) International application number: PCT/CN2015/088678
(87) International publication number: WO 2017/024639

(56) References cited:
- CN-A- 1 105 211
- CN-A- 1 530 033
- CN-A- 101 028 500
- CN-A- 101 036 760
- CN-A- 103 535 735
- DATABASE WPI Week 200975 Thomson Scientific, London, GB; AN 2009-Q76004 XP002787466, & CN 101 554 423 A (GUIZHOU NAT PHARMACY CO LTD) 14 October 2009 (2009-10-14)
- DATABASE WPI Week 201420 Thomson Scientific, London, GB; AN 2014-A16707 XP002787467, & CN 103 355 670 A (ZHAO C) 23 October 2013 (2013-10-23)
- DATABASE WPI Week 201404 Thomson Scientific, London, GB; AN 2013-U35265 XP002787468, & CN 103 230 554 A (WANG Y) 7 August 2013 (2013-08-07)
- DATABASE WPI Week 200815 Thomson Scientific, London, GB; AN 2008-B95413 XP002787469, & CN 101 028 500 A (SUN X) 5 September 2007 (2007-09-05)
- DATABASE WPI Week 201572 Thomson Scientific, London, GB; AN 2015-54176A XP002787470, & CN 104 771 567 A (ZHOU G) 15 July 2015 (2015-07-15)

## Description

### TECHNICAL FIELD

The present invention relates to the technical fields of medicines, health products, food and the like, and in particular to a Chinese medicine agent for use in relieving alcoholic intoxication, as specified in the claims.

### BACKGROUND ART

Alcoholic intoxication refers to a phenomenon that occurs when a person is out of control after drinking too much alcohol. Alcoholic intoxication can be generally divided into two types of severe alcoholic intoxication and mild alcoholic intoxication. There are the following symptoms for severe alcoholic intoxication: unsteady walking after drinking, allophasis, headache, vomiting and the like.

China is not only a country of long history of wine culture, but also a country of large production and consumption of wine beverages. With a significant improvement of material living standards of people in China, there has been an increase in the consumption of alcoholic (ethanol) beverages. In recent years, the incidence of adult alcoholism also increased accordingly. It is well-known that long-term alcohol drinking can cause systemic damage of multiple systems, and especially cause severe damages to gastrointestinal tracts and livers, and alcohol can directly or indirectly cause chronic gastritis, peptic ulcer and alcoholic cirrhosis. Acute alcoholism and alcohol dependence are one of the social problems today, and in case of unlimited excessive drinking or even alcoholism, in severe cases, a person will die from over-inhibition of the central nervous system by ethanol. Therefore, the alcoholism is harmful to the body.

### DISCLOSURE TO THE INVENTION

### TECHNICAL PROBLEMS

With the progress of the society, the development of science and technology and the improvement of people's living standards, people tend to have a lot of dinner parties for social entertainment, business contacts, family and friends and the like, and drinking is absolutely essential. High alcohol concentration in the body will cause alcoholic intoxication, alcoholism and even death. At present, there are many alcoholic intoxication relieving drugs available in the market. However, most of these alcoholic intoxication relieving drugs mainly focus on the protection of liver function while neglecting the damage caused by alcohol to other organs of the human body and fail to provide timely health care for other organs while maintaining the liver.

At present, there are many alcoholic intoxication relieving drugs in China, which mainly focus on traditional Chinese medicine or the combination of Chinese and Western medicines, but there are disadvantages such as high cost and unsatisfactory effects.

### SOLUTIONS TO THE PROBLEMS

### TECHNICAL SOLUTIONS

In view of the above defects and problems in the prior art, an objective of the embodiment of the present invention is to provide an alcoholic intoxication relieving agent with better reliability. Under the guidance of Chinese medicine theory, the effects of synergistic prevention of alcoholic intoxication and alcoholic intoxication relieving are achieved by changing the process of ethanol absorption and metabolism, the symptoms of unstable walking after drinking, allophasis, headache and vomiting are alleviated, and the damage of alcohol to various organs of the body is reduced.

To achieve the above objective, the embodiments of the present invention adopt the following technical solution.

The Chinese medicine agent for use in relieving alcoholic intoxication is characterized by being prepared from Fructus Crataegi, Folium Nelumbinis, Folium Mori, Herba Taraxaci, Herba Lophatheri, Herba Spirodelae, and Folium Citri tangerinae, wherein the Fructus Crataegi has effects of invigorating the spleen and promoting circulation of QI; the Folium Nelumbinis has effects of invigorating the spleen and ascending YIN; the Folium Mori has effects of clearing away lung-heat and moistening dryness; the Herba Lophatheri has effects of clearing away heat and relieving restlessness; the Herba Taraxaci has effects of clearing away heat and removing toxic substances; the Herba Spirodelae has effects of expelling pathogenic wind and promoting diuresis; and the Folium Citri tangerinae has effects of dispersing the stagnated liver-QI and promoting circulation of QI,

and the Chinese medicine alcoholic intoxication relieving agent is soaked in water with a single dosage for drinking: 10 g to 100 g of Fructus Crataegi, 1 g to 10 g of Folium Nelumbinis, 1 g to 10 g of Folium Mori, 1 g to 15 g of Herba Taraxaci, 1 g to 10 g of Herba Lophatheri, 1 g to 10 g of Herba Spirodelae and 1 g to 10 g of Folium Citri tangerinae.

As a preferred selection from above technical solutions, the Chinese medicine alcoholic intoxication relieving agent is prepared from the following components with 1 g by weight as one part: 9 parts of Fructus Crataegi, 1 part of Folium Nelumbinis, 3 parts of Folium Mori, 2 parts of Herba Taraxaci, 5 parts of Herba Lophatheri, 6 parts of Herba Spirodelae and 4 parts of Folium Citri tangerinae.

As a preferred selection from above technical solutions, the Chinese medicine alcoholic intoxication relieving agent is prepared in forms of decoctions, pills, capsules and oral liquids.

As a preferred selection from above technical solutions, the Chinese medicine alcoholic intoxication relieving agent is prepared by mixing extracts of Fructus Crataegi, Folium Nelumbinis, Folium Mori, Herba Taraxaci, Herba Lophatheri, Herba Spirodelae and Folium Citri tangerinae, i.e., alcoholic intoxication relieving enzymes according to a mass portion of 11: 22: 22: 11: 23: 11.

As a preferred selection from above technical solutions, the Chinese medicine alcoholic intoxication relieving agent is taken 20 to 30 minutes before drinking wine.

According to the Chinese medicine alcoholic intoxication relieving agent provided by the embodiments of the present invention, in order to reduce the damage of alcohol to the body from the source, as compared with the traditional alcoholic intoxication relieving formula, the invention has effects of invigorating the spleen and stomach as well as preserving the liver, such that the liver cannot be damaged by alcoholism through the spleen and stomach. Through human feeding trails (see the following table), the peak concentration after acute alcohol intake can be reduced remarkably, the phenomenon of alcohol metabolism lag does not occur, and the metabolism of the organism to alcohol is accelerated.

### BENEFICIAL EFFECTS OF THE INVENTION

### BENEFICIAL EFFECTS

The present invention has the following beneficial effects: with acceleration of alcohol metabolism, the alcohol concentration in blood can be reduced remarkably after heavy drinking, thereby reducing a variety of short-term discomforts after heavy drinking. The alcohol metabolism can be effectively accelerated to achieve the purpose of relieving alcoholic intoxication, and protecting people's kidneys, liver and other organs, and greatly reduce the damage of alcohol to the human body. The Chinese medicine alcoholic intoxication relieving agent disclosed by the present invention is low in economic cost and very simple in preparation at the same time. The Chinese medicine alcoholic intoxication relieving agent can be prepared into capsules, tablets, honeyed pills, oral liquids and other health products, medicines or food.

### OPTIMAL EMBODIMENTS IMPLEMENTING THE INVENTION

### OPTIMAL EMBODIMENTS OF THE INVENTION

### EMBODIMENTS IMPLEMENTING THE INVENTION

### EMBODIMENTS OF THE INVENTION

The technical solution of the present invention is described clearly and completely as below. It is apparent that the described embodiments are merely a part of embodiments of the present invention, rather than all of the embodiments. Based on the embodiments of the present invention, all of the other embodiments made by those ordinary skilled in the art without paying creative labor falls into the protection scope of the present invention.

### Embodiment 1

In an embodiment of the present invention, the Chinese medicine alcoholic intoxication relieving agent is prepared from Fructus Crataegi, Folium Nelumbinis, Folium Mori, Herba Taraxaci, Herba Lophatheri, Herba Spirodelae and Folium Citri tangerinae according to 10 g to 100 g of Fructus Crataegi, 1 g to 10 g of Folium Nelumbinis, 1 g to 10 g of Folium Mori, 1 g to 15 g of Herba Taraxaci, 1 g to 10 g of Herba Lophatheri, 1 g to 10 g of Herba Spirodelae and 1 g to 10 g of Folium Citri tangerinae. Sixteen volunteers are randomly divided into two groups of a traditional Chinese medicine group and a blank group. After the two groups are fasting for 4 hours, each volunteer in the Chinese medicine group drinks 100ml of the Chinese medicine alcoholic intoxication relieving agent beverage of the embodiment of the present invention, and each volunteer in the blank group drinks 100ml of normal saline; after drinking the Chinese medicine agent and the normal saline for 40 minutes, volunteers in the two groups drink 50ml of 42-degreeErguotou; after 20 minutes of drinking, a breathalyzer is adopted to detect the alcohol content, and the results are as follows.

**[Table 1]**

| Unit mg/100ml | Chinese Medicine Group | Blank Group |
|---|---|---|
| 1 | 14 | 44 |
| 2 | 12 | 42 |
| 3 | 15 | 45 |
| 4 | 9 | 40 |
| 5 | 17 | 50 |
| 6 | 10 | 47 |
| 7 | 11 | 49 |
| 9 | 13 | 43 |

The data results show that after each volunteer in the Chinese medicine group drinks 100ml of traditional Chinese medicine alcoholic intoxication relieving agent and each volunteer in the blank group drinks 100ml of normal saline, the alcohol concentration in the body of each volunteer in the Chinese medicine group is significantly lower than that in the body of each volunteer in the blank group.

## Claims

1. A Chinese medicine agent for use in relieving alcoholic intoxication, **characterized by** being prepared from Fructus Crataegi, Folium Nelumbinis, Folium Mori, Herba Taraxaci, Herba Lophatheri, Herba Spirodelae and Folium Citri tangerinae, wherein the Fructus Crataegi has effects of invigorating the spleen and promoting the circulation of QI; the Folium Nelumbinis has effects of invigorating the spleen and ascending YIN; the Folium Mori has effects of clearing away lung-heat and moistening dryness; the Herba Lophatheri has effects of clearing away heat and relieving restlessness; the Herba Taraxaci has effects of clearing away heat and removing toxic substances; the Herba Spirodelae has effects of expelling pathogenic wind and promoting diuresis; and the Folium Citri tangerinae has effects of dispersing the stagnated liver-QI and promoting circulation of QI, the Chinese medicine alcoholic intoxication relieving agent being soaked in water with a single dosage for drinking: 10 g to 100 g of Fructus Crataegi, 1 g to 10 g of Folium Nelumbinis, 1 g to 10 g of Folium Mori, 1 g to 15 g of Herba Taraxaci, 1 g to 10 g of Herba Lophatheri, 1 g to 10 g of Herba Spirodelae and 1 g to 10 g of Folium Citri tangerinae.

2. The Chinese medicine agent for use according to claim 1, **characterized in that** the Chinese medicine alcoholic intoxication relieving agent is prepared in the orally-administrated forms, such as decoctions, pills, capsules and oral liquids.

3. The Chinese medicine agent for use according to claim 1, **characterized in that** the Chinese medicine alcoholic intoxication relieving agent is prepared by mixing extracts of Fructus Crataegi, Folium Nelumbinis, Folium Mori, Herba Taraxaci, Herba Lophatheri, Herba Spirodelae and Folium Citri tangerinae, i.e., alcoholic intoxication relieving enzymes according to 10 g to 100 g of Fructus Crataegi, 1 g to 10 g of Folium Nelumbinis, 1 g to 10 g of Folium Mori, 1 g to 15 g of Herba Taraxaci, 1 g to 10 g of Herba Lophatheri, 1 g to 10 g of Herba Spirodelae and 1 g to 10 g of Folium Citri tangerinae.

4. The Chinese medicine agent for use according to claim 1, **characterized in that** the Chinese medicine alcoholic intoxication relieving agent is taken 20 to 30 minutes before drinking wine.

## Patentansprüche

1. Mittel aus der chinesischen Medizin zur Verwendung bei der Linderung von Alkoholintoxikation, **dadurch gekennzeichnet, dass** es aus Fructus Crataegi, Folium Nelumbinis, Folium Mori, Herba Taraxaci, Herba Lophatheri, Herba Spirodelae and Folium Citri tangerinae hergestellt ist, wobei Fructus Crataegi die Effekte aufweist, dass die Milz belebt wird und der Fluss des Qi gefördert wird; Folium Nelumbinis die Effekte aufweist, dass die Milz belebt wird und das Yin ansteigt; Folium Mori die Effekte aufweist, dass Lungenhitze verschwindet und Trockenheit befeuchtet wird; Herba Lophatheri die Effekte aufweist, dass Hitze verschwindet und Rastlosigkeit gelindert wird; Herba Taraxaci die Effekte aufweist, dass Hitze verschwindet und toxische Stoffe entfernt werden; Herba Spirodelae die Effekte aufweist, dass pathogener Wind ausgetrieben wird und die Diurese gefördert wird; und Folium Citri tangerinae die Effekte aufweist, dass sich das stockende Leber-Qi auflöst und der Fluss des Qi gefördert wird, wobei man das Mittel zur Linderung von Alkoholintoxikation aus der chinesischen Medizin mit folgender Einzeldosierung in Wasser zum Trinken ziehen lässt: 10 g bis 100 g Fructus Crataegi, 1 g bis 10 g Folium Nelumbinis, 1 g bis 10 g Folium Mori, 1 g bis 15 g Herba Taraxaci, 1 g bis 10 g Herba Lophatheri, 1 g bis 10 g Herba Spirodelae und 1 g bis 10 g Folium Citri tangerinae.

2. Mittel aus der chinesischen Medizin zur Verwendung nach Anspruch 1, **dadurch gekennzeichnet, dass** das Mittel aus der chinesischen Medizin zur Linderung von Alkoholintoxikation in oralen Verabreichungsformen wie Sud, Pillen, Kapseln und Oralflüssigkeiten hergestellt ist.

3. Mittel aus der chinesischen Medizin zur Verwendung nach Anspruch 1, **dadurch gekennzeichnet, dass** das Mittel aus der chinesischen Medizin zur Linderung von Alkoholintoxikation durch das Mischen von Extrakten von Fructus Crataegi, Folium Nelumbinis, Folium Mori, Herba Taraxaci, Herba Lophatheri, Herba Spirodelae und Folium Citri tangerinae, d. h. Alkoholintoxikation lindernden Enzymen, die 10 g bis 100 g Fructus Crataegi, 1 g bis 10 g Folium Nelumbinis, 1 g bis 10 g Folium Mori, 1 g bis 15 g Herba Taraxaci, 1 g bis 10 g Herba Lophatheri, 1 g bis 10 g Herba Spirodelae und 1 g bis 10 g Folium Citri tangerinae entsprechen, hergestellt ist.

4. Mittel aus der chinesischen Medizin zur Verwendung nach Anspruch 1, **dadurch gekennzeichnet, dass** das Mittel aus der chinesischen Medizin zur Linderung von Alkoholintoxikation 20 bis 30 Minuten vor dem Trinken von Wein eingenommen wird.

## Revendications

1. Agent de médecine chinoise pour une utilisation dans le soulagement de l'intoxication alcoolique, **caractérisé par le fait qu'**il est préparé à partir de Fructus Crataegi, Folium Nelumbinis, Folium Mori, Herba Taraxaci, Herba Lophatheri, Herba Spirodelae et de Folium Citri tangerinae, le Fructus Crataegi ayant pour effets de tonifier la rate et de promouvoir la circulation du Qi ; le Folium Nelumbinis ayant pour effets de tonifier la rate et de faire remonter le Yin ; le Folium Mori ayant pour effets de débarrasser de la chaleur pulmonaire et d'humidifier la sécheresse ; l'Herba Lophatheri ayant pour effets de débarrasser de la chaleur du poumon et de soulager l'agitation ; l'Herba Taraxaci ayant pour effets de débarrasser de la chaleur du poumon et d'éliminer des substances toxiques ; l'Herba Spirodelae ayant pour effets d'expulser le vent pathogène et de promouvoir la diurèse ; et le Folium Citri tangerinae possédant des effets de dispersion du foie-QI ayant stagné et de promotion de la circulation du Qi, l'agent de médecine chinoise de soulagement de l'intoxication alcoolique étant trempé dans l'eau avec un dosage unique à boire : 10 g à 100 g de Fructus Crataegi, 1 g à 10 g de Folium Nelumbinis, 1 g à 10 g de Folium Mori, 1 g à 15 g d'Herba Taraxaci, 1 g à 10 g d'Herba Lophatheri, 1 g à 10 g d'Herba Spirodelae et 1 g à 10 g de Folium Citri tangerinae.

2. Agent de médecine chinoise pour une utilisation selon la revendication 1, **caractérisé en ce que** l'agent de médecine chinoise de soulagement de l'intoxication alcoolique est préparé en des formes d'administration orale, telles que des décoctions, des pilules, des capsules et des liquides par voie orale.

3. Agent de médecine chinoise pour une utilisation selon la revendication 1, **caractérisé en ce que** l'agent de médecine chinoise de soulagement de l'intoxication alcoolique est préparé en mélangeant des extraits de Fructus Crataegi, Folium Nelumbinis, Folium Mori, Herba Taraxaci, Herba Lophatheri, Herba Spirodelae et de Folium Citri tangerinae, c'est-à-dire, des enzymes de soulagement de l'intoxication alcoolique selon 10 g à 100 g de Fructus Crataegi, 1 g à 10 g de Folium Nelumbinis, 1 g à 10 g de Folium Mori, 1 g à 15 g d'Herba Taraxaci, 1 g à 10 g d'Herba Lophatheri, 1 g à 10 g d'Herba Spirodelae et 1 g à 10 g de Folium Citri tangerinae.

4. Agent de médecine chinoise pour une utilisation selon la revendication 1, **caractérisé en ce que** l'agent de médecine chinoise de soulagement de l'intoxication alcoolique est pris 20 à 30 minutes avant de boire du vin.
